(19) Europäisches Patentamt / European Patent Office / Office européen des brevets

(11) **EP 4 217 335 B1**

(12) **FASCICULE DE BREVET EUROPEEN**

(45) Date de publication et mention de la délivrance du brevet:
**05.03.2025   Bulletin 2025/10**

(21) Numéro de dépôt: **21770274.5**

(22) Date de dépôt: **13.09.2021**

(51) Classification Internationale des Brevets (IPC):
**C07C 45/00** (2006.01)   **C07C 47/06** (2006.01)
**C07C 1/207** (2006.01)   **C07C 11/167** (2006.01)
**C01B 3/22** (2006.01)   **B01J 8/06** (2006.01)

(52) Classification Coopérative des Brevets (CPC):
(C-Sets disponibles)
**B01J 8/067; C01B 3/22; C07C 1/2076;**
**C07C 45/002;** C01B 2203/0277; C01B 2203/0833;
C01B 2203/1076; C01B 2203/1082;
C01B 2203/1229; C07C 2521/08; C07C 2523/20
(Cont.)

(86) Numéro de dépôt international:
**PCT/EP2021/075135**

(87) Numéro de publication internationale:
**WO 2022/063620 (31.03.2022 Gazette 2022/13)**

(54) **PROCÉDÉ DE DESHYDROGENATION DE L'ÉTHANOL EN REACTEUR MULTITUBULAIRE**

VERFAHREN ZUR DEHYDRIERUNG VON ETHANOL IN EINEM MEHRROHRREAKTOR

PROCESS FOR THE DEHYDROGENATION OF ETHANOL IN A MULTITUBULAR REACTOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **25.09.2020   FR 2009759**

(43) Date de publication de la demande:
**02.08.2023   Bulletin 2023/31**

(73) Titulaires:
• **IFP Energies nouvelles**
  **92500 Rueil-Malmaison (FR)**
• **COMPAGNIE GENERALE DES ETABLISSEMENTS MICHELIN**
  **63000 Clermont-Ferrand (FR)**

(72) Inventeurs:
• **GABELLE, Jean-Christophe**
  **92852 RUEIL-MALMAISON CEDEX (FR)**
• **COUPARD, Vincent**
  **92852 RUEIL-MALMAISON CEDEX (FR)**
• **DASTILLUNG, Rejane**
  **92852 RUEIL-MALMAISON CEDEX (FR)**
• **MEJEAN, Mickael**
  **92852 RUEIL-MALMAISON CEDEX (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
**FR-A1- 3 090 632**

• **FILHO R.MACIEL ET AL: "A multitubular reactor for obtention of acetaldehyde by oxidation of ethyl alcohol", CHEMICAL ENGINEERING SCIENCE, vol. 47, no. 9-11, 1 June 1992 (1992-06-01), GB, pages 2571 - 2576, XP055810653, ISSN: 0009-2509, DOI: 10.1016/ 0009-2509(92)87095-8**

(52) Classification Coopérative des Brevets (CPC):
(Cont.)

C-Sets
**C07C 1/2076, C07C 11/167;**
**C07C 45/002, C07C 47/06**

**Description**

**Domaine de l'invention**

La présente invention se rapporte à un procédé de déshydrogénation catalytique de l'éthanol, mettant en particulier en œuvre au moins un réacteur multitubulaire à condensation d'un fluide caloporteur. La présente invention concerne également un procédé de production de butadiène à partir d'éthanol qui comprend comme première étape réactionnelle le procédé de déshydrogénation catalytique de l'éthanol en réacteur multitubulaire à condensation du fluide caloporteur pour produire de l'acétaldéhyde, suivi d'une seconde étape réactionnelle produisant du butadiène à partir d'un mélange d'éthanol et d'acétaldéhyde.

**Technique antérieure**

La réaction de déshydrogénation de l'éthanol en acétaldéhyde peut être une première étape réactionnelle dans plusieurs procédés, comme par exemple dans un procédé de conversion de l'éthanol en butadiène, connu en particulier sous le nom de procédé d'Ostromislensky et associé au procédé Lebedev. La réaction de déshydrogénation d'éthanol est une réaction équilibrée qui présente des taux de conversion de l'éthanol classiquement autour de 30%. C'est une réaction très endothermique ($\Delta$H réaction = 72,4 kJ/mol). Une technologie de réacteur permettant une compensation thermique adéquate est donc nécessaire.

Plus globalement, il existe plusieurs technologies de procédés industriels de déshydrogénation : le procédé Catofin® d'ABB, le procédé Oleflex™ d'UOP, le procédé Star Process® de Thyssenkrupp et le procédé PDH de Linde. Tous ces procédés industriels ont pour objectif la déshydrogénation d'alcanes légers en oléfines, en particulier la déshydrogénation du propane et butane en respectivement propylène et butène. Plus particulièrement, les procédés Catofin® et Oleflex™ mettent en œuvre des batteries de réacteurs adiabatiques en parallèle, respectivement à lits catalytiques fixes et à lits catalytiques mobiles. Le procédé PDH quant à lui utilise des réacteurs à lits fixes chauffés par l'extérieur par un combustible. Le procédé Star Process® réalise la réaction dans des tubes placés dans un four sur une charge diluée à la vapeur d'eau. Il semble que le choix de la technologie de réacteur dépend au moins en partie du catalyseur utilisé pour la déshydrogénation des alcanes mise en œuvre.

Les réacteurs multitubulaires, encore appelées réacteurs-échangeurs, peuvent être utilisés pour permettre un fonctionnement des procédés en conditions isothermes ou pseudo-isothermes. C'est le cas par exemple du procédé de déshydratation de l'isopropanol décrit dans le brevet US 5,227,563 de Mitsui Petrochemical. Dans ce brevet, l'isopropanol est déshydraté en propylène en présence d'alumine, entre 290°C et 320°C dans un réacteur tubulaire vertical, de 0,5 m de longueur et 25,4 mm de diamètre interne, pour obtenir des taux de conversion de l'isopropanol d'au moins 85% et une sélectivité en propylène de 79% ou plus.

La demande de brevet WO 2018/046515 décrit quant à elle un procédé de déshydratation de l'isobutanol en butène comprenant une étape de déshydratation et isomérisation simultanée, opérée dans des conditions isothermes ou pseudo-isothermes à une température de 300°C ou 350°C, en présence d'un catalyseur comprenant une zéolithe FER, dans un réacteur multitubulaire. Les taux de conversion de l'isobutanol obtenus par ce procédé sont de l'ordre de 100%, avec une sélectivité en butènes d'au moins 97%.

La demande FR 3 089 973 décrit enfin un procédé de déshydratation de l'éthanol en éthylène, dans un réacteur multitubulaire, en présence de zéolithe ZSM-5 et à une température d'entrée de la charge dans les tubes entre 420 et 430°C. Pour maintenir la température, des sels fondus à 470°C sont utilisés comme fluide caloporteur. Dans ces conditions, les taux de conversion d'éthanol atteints sont supérieurs à 99% avec une sélectivité en éthylène d'au moins 98%.

Parallèlement, il existe des travaux relativement à la conversion de l'éthanol en acétaldéhyde. Par exemple, la littérature décrit un procédé d'obtention d'acétaldéhyde par oxydation de l'éthanol en présence d'un catalyseur à base de fer et de molybdène, le procédé mettant en œuvre un réacteur multitubulaire particulier dont la calandre comprend plusieurs chambres (en particulier cinq chambres), chacune de ces chambres étant alimentée en un fluide refroidissant (Filho R.M. at al.: « A multitubular reactor for obtention of acetaldehyde by oxidation of ethyl alcohol », Chemical Engineering Science, vol.47, no. 9-11, 1 juin 1992, pages 2571-2576). Ainsi, malgré la forte exothermicité de la réaction d'oxydation, la température dans les tubes est contrôlée entre 180 et 240°C, ce qui permet de limiter les réactions secondaires et la formation de sous-produits.

Cependant, aucun de ces documents n'aborde la réaction de la déshydrogénation de l'éthanol en acétaldéhyde.

Le document FR3090632 divulgue un procédé de préparation de butadiène à partir d'éthanol, comprenant une première étape réactionnelle de déshydrogénation de l'éthanol en acétaldéhyde, effectuée à 275°C dans un réacteur multitubulaire et en présence d'un catalyseur à base de cuivre. Cependant, ce document ne donne aucune indication permettant une conversion optimale de l'éthanol en acétaldéhyde par déshydrogénation.

Un objectif de l'invention est donc de fournir un procédé de déshydrogénation de l'éthanol en acétaldéhyde, permettant d'atteindre une conversion de l'éthanol et une sélectivité en acétaldéhyde satisfaisantes, tout en évitant une désactivation prématurée du catalyseur et en limitant une consommation importante d'utilités telles que la vapeur d'eau et en minimisant les coûts d'investissement et de fonctionnement.

**Résumé de l'invention**

L'invention concerne un procédé de déshydrogénation de l'éthanol en acétaldéhyde, comprenant une étape de déshydrogénation d'une charge comprenant de l'éthanol, ladite étape de déshydrogénation mettant en œuvre une section réactionnelle comprenant au moins un réacteur multitubulaire qui comprend un ou une pluralité de tubes et une calandre,

ledit (ou lesdits) tube(s) comprenant chacun au moins un lit fixe d'au moins un catalyseur de déshydrogénation, ladite charge alimentant ledit (ou lesdits) tube(s) sous forme gazeux, à une température d'entrée de ladite charge supérieure ou égale à 240°C, à une pression d'entrée de ladite charge entre 0,1 et 1,0 MPa, et à une vitesse spatiale horaire en poids (PPH) de la charge en entrée entre 2 et 15 h$^{-1}$,

un fluide caloporteur circulant dans ladite calandre de sorte que ledit fluide caloporteur est introduit dans ladite calandre sous forme gazeux et est, en sortie de calandre, au moins en partie sous forme liquide,

ledit fluide caloporteur étant introduit dans la calandre à un débit tel que le ratio du débit pondéral dudit fluide caloporteur en entrée de calandre par rapport au débit pondéral de ladite charge en entrée du (ou des) tubes est supérieur ou égal à 1,0,

ledit fluide caloporteur étant introduit dans la calandre à une température d'entrée du fluide caloporteur supérieure ou égale à 260°C, et inférieure ou égale à 400°C, et à une pression d'entrée du fluide caloporteur supérieure ou égale à 0,10 MPa, et inférieure ou égal à 1,10 MPa ;

ledit procédé produisant un effluent de déshydrogénation comprenant au moins de l'acétaldéhyde, de l'hydrogène et de l'éthanol non converti.

La présente invention présente l'avantage de compenser l'endothermicité de la réaction de déshydrogénation de l'éthanol ($\Delta$H réaction = 72,4 kJ/mol) en maintenant la température du milieu réactionnel dans une gamme de températures adaptées à la réaction de déshydrogénation, en particulier à une valeur supérieure ou égale à 230°C, de préférence supérieure ou égale à 240°C, de manière préférée supérieure ou égale à 250°C. La présente invention met en œuvre un réacteur de type réacteur - échangeur et selon un mode particulier de condensation du fluide caloporteur. L'enthalpie de changement de phase du fluide caloporteur peut ainsi être utilisée pour apporter la chaleur nécessaire aux besoins énergétiques de la réaction de déshydrogénation de l'éthanol. La présente invention permet également d'avoir une température homogène en paroi extérieur des tubes à l'intérieur desquels la réaction est mise en œuvre.

La présente invention permet ainsi d'atteindre des taux de conversion de l'éthanol satisfaisants et une sélectivité en acétaldéhyde élevée, en particulier des taux de conversion de l'éthanol supérieurs ou égaux à 25 de préférence de l'ordre de 35% et une sélectivité en acétaldéhyde supérieure à 90%.

Un autre avantage de l'invention réside dans le fait que la compensation thermique est obtenue sans ajout dans la charge d'un diluant qui apparait nécessaire, notamment lorsque des réacteurs adiabatiques sont utilisés, pour « tamponner thermiquement » la chute de température induite par l'endothermicité de la réaction en transmettant sa chaleur sensible à la réaction. Le diluant classiquement utilisé comme « tampon thermique » est la vapeur d'eau. Or une teneur en eau trop importante dans le réacteur, par exemple une teneur en eau supérieure à 20% poids par rapport au poids total de la charge, peut être néfaste au catalyseur de déshydrogénation et engendrer sa désactivation précoce. Ainsi, le procédé de déshydrogénation de l'éthanol de la présente invention ne demande aucun ajout de diluant et permet alors de limiter le risque de désactivation précoce du catalyseur de déshydrogénation.

La présente invention propose également l'avantage de pouvoir déshydrogéner des charges éthanol de compositions et d'origines très diverses, par exemple pouvant comprendre de l'eau en mélange avec l'éthanol. En particulier, la présente invention peut en effet s'appliquer à des charges éthanol produites à partir de sources renouvelables issues de la biomasse, souvent appelées "bioéthanol. Elle peut également s'appliquer à un effluent riche en éthanol par exemple obtenu après traitement d'un effluent réactionnel issu en particulier de la conversion de l'éthanol en butadiène et avantageusement recyclé vers la section réactionnelle, ladite section réactionnelle comprenant notamment une étape de déshydrogénation de l'éthanol comme décrit dans le brevet FR 3 026 100. Plus particulièrement, la présente invention permet la déshydrogénation de l'effluent riche en éthanol obtenu à l'étape E1) du brevet FR 3 026 100 et qui peut comprendre par exemple jusqu'à 18% poids d'eau.

Par ailleurs, le fait d'éviter l'ajout d'un diluant dans la charge permet avantageusement de limiter la taille des équipements et le nombre d'étapes ultérieures de séparation et par conséquent de minimiser les coûts d'investissement et de fonctionnement du procédé, en particulier par rapport à un procédé de déshydrogénation de l'éthanol nécessitant une dilution à la vapeur d'eau.

La présente invention présente encore un autre avantage : celui d'éviter la multiplication du nombre de lits catalytiques nécessaire. En effet, avec la technologie des réacteurs adiabatiques, une succession de lits fixes de catalyseurs, intercalés d'échangeurs de chaleur, est nécessaire pour compenser l'endothermie de la réaction et atteindre une conversion de l'éthanol de l'ordre de 30%. Au contraire, la présente invention, qui propose d'utiliser un réacteur multitubulaire couplé au mode particulier de condensation d'un fluide caloporteur adapté, permet d'atteindre les taux de conversion visés (entre 25% et 35%, voire entre 30 et 35%) et une sélectivité en acétaldéhyde élevée (supérieure ou égale à 90%), tout en limitant les coûts d'investissement et de fonctionnement du procédé.

## Description des modes de l'invention

Selon la présente invention, les expressions « compris entre ... et ... » et « entre .... et ... » sont équivalentes et signifient que les valeurs limites de l'intervalle sont incluses dans la gamme de valeurs décrite. Si tel n'était pas le cas et que les valeurs limites n'étaient pas incluses dans la gamme décrite, une telle précision sera apportée par la présente invention.

Dans le sens de la prése nte invention, les différentes plages de paramètres pour une étape donnée tels que les plages de pression et les plages de température peuvent être utilisées seules ou en combinaison. Par exemple, dans le sens de la présente invention, une plage de valeurs préférées de pression peut être combinée avec une plage de valeurs de température plus préférées.

Dans la suite, des modes de réalisation particuliers et/ou préférés de l'invention peuvent être décrits. Ils pourront être mis en œuvre séparément ou combinés entre eux, sans limitation de combinaison lorsque c'est techniquement réalisable.

L'invention concerne ainsi un procédé de déshydrogénation d'éthanol en acétaldéhyde, comprenant, de préférence consistant en, une étape de déshydrogénation d'une charge comprenant de l'éthanol, de préférence au moins 50% poids d'éthanol, préférentiellement au moins 70% poids d'éthanol, de manière préférée au moins 80% poids d'éthanol, et éventuellement de l'eau, dans lequel :

- ladite étape de déshydrogénation met en œuvre une section réactionnelle comprenant au moins un réacteur multitubulaire qui comprend un ou une pluralité de tubes, de préférence une pluralité de tubes et une calandre, ledit (ou lesdits) tube(s) étant très avantageusement en acier de tout type de préférence en acier allié et de manière préférée en acier inoxydable, ayant de préférence une longueur entre 1,0 et 6,0 m, de manière préférée entre 2,0 et 3,0 m, de préférence un diamètre interne entre 30,0 et 60,0 mm, préférentiellement entre 40,0 et 50,0 mm, et de préférence une épaisseur de paroi de tube entre 1,5 et 5,0 mm, préférentiellement entre 2,0 et 4,0 mm et de manière préférée entre 2,2 et 3,2 mm,
- chaque tube comprend au moins un lit fixe comprenant au moins un catalyseur de déshydrogénation, ledit catalyseur de déshydrogénation comprenant de préférence au moins l'élément cuivre sur un support inorganique de manière préférée de la silice, ledit catalyseur étant très avantageusement sous forme de particules de diamètre moyen équivalent entre 0,5 et 10,0 mm, de préférence entre 1,0 et 5,0 mm,
- ladite charge alimente ledit (ou lesdits) tube(s) sous forme gazeux, à une température d'entrée de ladite charge dans le (ou les) tube(s) supérieure ou égale à 240°C, de préférence entre 240°C et 350°C, préférentiellement entre 250°C et 300°C, de manière préférée entre 260°C et 290°C, à une pression d'entrée de ladite charge dans le (ou les) tube(s) entre 0,1 et 1,0 MPa, de préférence entre 0,2 et 0,5 MPa, préférentiellement entre 0,3 et 0,4 MPa, et à une vitesse spatiale horaire (PPH) de la charge en entrée du réacteur multitubulaire entre 2 et 15 $h^{-1}$, de préférence entre 2 et 10 $h^{-1}$,
- un fluide caloporteur circule dans ladite calandre, de préférence à co-courant de la charge dans le (ou les) tube(s), de sorte que ledit fluide caloporteur est introduit dans ladite calandre sous forme gazeux et est, en sortie de calandre, au moins en partie sous forme liquide,
- ledit fluide caloporteur est introduit dans la calandre dudit réacteur multitubulaire à un débit tel que le ratio du débit pondéral dudit fluide caloporteur en entrée de calandre par rapport au débit pondéral de ladite charge en entrée desdits tubes est supérieur ou égal à 1,0, de préférence supérieure ou égale à 1,5, et avantageusement inférieur ou égal à 10,0, de préférence inférieur ou égal à 5,0, de manière préférée inférieur ou égal à 2,0,
- ledit fluide caloporteur est introduit dans la calandre dudit réacteur multitubulaire à une température d'entrée du fluide caloporteur supérieure ou égale à 260°C, de préférence supérieure ou égale à 270°C, de manière préférée supérieure ou égale à 290°C, et inférieure ou égale à 400°C, de préférence inférieure ou égale à 380°C, et à une pression d'entrée du fluide caloporteur supérieure ou égale à 0,10 MPa, de préférence supérieure ou égale à 0,13 MPa, de manière préférée supérieure ou égale à 0,20 MPa, et inférieure ou égal à 1,10 MPa, de manière préférée inférieure ou égale à 0,85 MPa ;

ledit procédé produisant un effluent de déshydrogénation comprenant au moins de l'acétaldéhyde, de l'hydrogène, et de l'éthanol non converti.

**Charge**

Conformément à l'invention, la charge traitée dans le procédé de déshydrogénation est une charge comprenant de l'éthanol. De préférence, ladite charge comprend au moins 50% poids d'éthanol, préférentiellement au moins 70% poids d'éthanol, de manière préférée au moins 80% poids d'éthanol.

La charge du procédé de déshydrogénation peut en outre comprendre éventuellement de l'eau, de préférence à une teneur inférieure à 50% poids, préférentiellement inférieure à 30% poids, de manière préférée inférieure à 20% poids, par exemple entre 1 et 20% poids d'eau par rapport au poids total de la charge .

La charge, en particulier comprenant moins de 50% poids d'éthanol, peut être concentrée, préalablement au procédé de l'invention, par tous moyens connus de l'Homme du métier, par exemple par distillation, par absorption, par pervaporation, par extraction avec un solvant.

Ladite charge peut comprendre des impuretés, en plus de l'eau, comme par exemple du butanol, de préférence à une teneur inférieure ou égale à 10 % poids, préférentiellement inférieure ou égale à 5 % poids, de manière préférée inférieure ou égale à 2 % poids, par rapport au poids totale de ladite charge.

La charge traitée dans le procédé selon l'invention est éventuellement obtenue par un procédé de synthèse d'alcool à partir de ressources fossiles telles que par exemple à partir du charbon, du gaz naturel ou des déchets carbonés.

De préférence, la charge provient avantageusement de ressources non fossiles. Elle peut être obtenue à partir de sources renouvelables issues de la biomasse, souvent appelée "bioéthanol". Le bioéthanol est une charge produite par voie biologique, de préférence par fermentation de sucres issus par exemple des cultures de plantes sucrières comme la canne à sucre (saccharose, glucose, fructose, et sucrose), des betteraves, ou encore des plantes amylacées (amidon) ou de la biomasse lignocellulosique ou de cellulose hydrolysée (glucose majoritaire et xylose, galactose), contenant des quantités variables d'eau. Pour une description plus complète des procédés fermentaires classiques, on peut se reporter à l'ouvrage 'Les Biocarburants, État des lieux, perspectives et enjeux du développement', Daniel Ballerini, Editions Technip, 2006.

Ladite charge peut également avantageusement être obtenue à partir de gaz de synthèse.

Ladite charge peut encore avantageusement être obtenue par hydrogénation des acides ou esters correspondants. Dans ce cas, l'acide acétique ou les esters acétiques sont avantageusement hydrogénés à l'aide d'hydrogène en éthanol. L'acide acétique peut avantageusement être obtenu par carbonylation du méthanol ou par fermentation des carbohydrates.

Ladite charge peut aussi être un effluent éthanol, obtenu après traitement (en particulier après des étapes de séparation et purification) d'un effluent issu d'un procédé de conversion d'éthanol en butadiène. Plus particulièrement, la charge du procédé de déshydrogénation peut être un effluent éthanol comprenant majoritairement de l'éthanol, c'est-à-dire comprenant au moins 50% poids, de préférence au moins 70% poids, préférentiellement au moins 80% poids d'éthanol, et obtenu après traitement d'un effluent réactionnel issu de la conversion de l'éthanol en butadiène, ledit effluent éthanol étant avantageusement recyclé vers la section réactionnelle qui comprend en particulier une étape de déshydrogénation de l'éthanol. Selon un mode de réalisation très particulier de l'invention, la charge comprenant de l'éthanol est un effluent riche en éthanol, avantageusement obtenu à l'issue d'une étape de traitement des effluents d'un procédé de conversion de l'éthanol en butadiène, comme par exemple l'effluent riche en éthanol avantageusement obtenu à l'issue de l'étape E1) du procédé décrit dans le brevet FR 3 026 100. Cet effluent riche en éthanol peut comprendre notamment jusqu'à 18% poids d'eau.

**Étape de déshydrogénation**

Conformément l'invention, le procédé de déshydrogénation comprend au moins une étape de déshydrogénation de ladite charge comprenant de l'éthanol, de manière à produire un effluent de déshydrogénation comprenant avantageusement au moins de l'acétaldéhyde, de l'hydrogène et classiquement de l'éthanol non converti. Ledit effluent de déshydrogénation obtenu peut également comprendre de l'eau, en particulier lorsque la charge comprend elle-même de l'eau. Il peut aussi comprendre des impuretés, en particulier déjà présentes dans la charge, et/ou des co-produits notamment générés lors de la réaction de déshydrogénation.

Ladite étape de déshydrogénation met en œuvre une section réactionnelle comprenant au moins un réacteur multitubulaire dans lequel la réaction de déshydrogénation a lieu. La section réactionnelle peut comprendre au moins deux réacteurs multitubulaires, et de préférence moins de dix réacteurs multitubulaires. De préférence, la section réactionnelle comprend deux réacteurs multitubulaires, l'un étant en fonctionnement, c'est-à-dire alimenté en charge et mettant en œuvre la réaction de déshydrogénation, l'autre étant en mode régénération-remplacement. L'expression « en mode régénération-remplacement » signifie que le réacteur multitubulaire n'est pas alimenté en charge comprenant l'éthanol et que le catalyseur est en cours de régénération ou de chargement, ou encore est régénéré et/ou chargé et est prêt à fonctionner (c'est-à-dire en attente de fonctionnement).

Avantageusement, chaque réacteur multitubulaire comprend un ou une pluralité de tubes et une calandre. La réaction de déshydrogénation a lieu avantageusement dans le (ou les) tube(s) du (ou des) réacteur(s) multitubulaire(s) avantageusement en fonctionnement. Dans la suite de l'exposé, le(les) tube(s) du(des) réacteur(s) multitubulaire(s) peu(ven)t également être appelé(s) tube(s) réactionnel(s). La calandre est l'enveloppe, typiquement cylindrique, du réacteur à l'intérieur de laquelle se situent le ou les tubes, de préférence parallèles entre eux et aux parois de la calandre lorsqu'il y a plusieurs tubes, et dans laquelle circule un fluide caloporteur. La calandre peut également comprendre une ou plusieurs chicanes ou tout autre système, réparti(es) de préférence de manière homogène dans la calandre, pour permettre une bonne diffusion et homogénéisation du fluide caloporteur et donc une bonne répartition de la chaleur. La calandre et les tubes peuvent avoir un design particulier ou une texture particulière permettant de favoriser la condensation et/ou l'évacuation du fluide caloporteur.

Selon l'invention, chaque tube comprend au moins un lit fixe comprenant au moins un catalyseur de déshydrogénation. De préférence, chaque tube contient un lit fixe d'un catalyseur de déshydrogénation. De manière préférée, ledit catalyseur de déshydrogénation comprend au moins l'élément cuivre, et éventuellement l'élément chrome, sur un support inorganique de manière préférée la silice. Très avantageusement, le catalyseur de déshydrogénation est sous forme de particules de diamètre moyen équivalent entre 0,5 et 10,0 mm, de préférence entre 1,0 et 5,0 mm. Selon l'invention, Le diamètre moyen équivalent définit un diamètre moyen équivalent en surface, avantageusement déterminé par la méthode de diffraction laser et le diamètre moyen équivalent des particules correspond avantageusement au diamètre moyen de sphères ayant la même surface spécifique que lesdites particules. Par exemple, le catalyseur de déshydrogénation peut être le catalyseur Octolyst® 2001 ou Octolyst® 2009 commercialisé par Evonik.

De préférence, le(ou les) réacteur(s) multitubulaire(s) comprend(comprennent) une pluralité de tubes dans la calandre, de préférence au moins 100 tubes, préférentiellement au moins 1000 tubes, de manière préférée au moins 2000 tubes. Généralement, les réacteurs multitubulaires comprennent jusqu'à 20 000 tubes, de préférence jusqu'à 10 000 tubes. Par exemple, chaque réacteur multitubulaire peut contenir entre 5 000 et 6 000 tubes.

Avantageusement, le(ou les) tube(s) réactionnel(s) ont une longueur de préférence comprise entre 1,0 et 6,0 m, de manière préférée entre 2,0 et 3,0 m. Lorsque le réacteur multitubulaire comprend une pluralité de tubes, tous les tubes dudit réacteur multitubulaire présentent avantageusement la même longueur, à la précision près due notamment à la fabrication et l'usinage des tubes. Le diamètre interne de chaque tube réactionnel est de préférence compris entre 30,0 et 60,0 mm, préférentiellement entre 40,0 et 50,0 mm. Avantageusement, le(ou les) tube(s) réactionnel(s) présente(nt) une épaisseur de paroi de préférence entre 1,5 et 5,0 mm, préférentiellement entre 2,0 et 4,0 mm et de manière préférée entre 2,2 et 3,2 mm. Ainsi, le diamètre nominal, ou diamètre externe, d'un tube peut varier entre 33 et 70 mm, de préférence entre 44 et 56 mm. Le dimensionnement spécifique du(des) tube(s) réactionnel(s), en particulier la longueur, le diamètre interne et l'épaisseur de la paroi, est avantageusement adapté aux pressions exercées coté tube (c'est-à-dire à l'intérieur du(des) tube(s)) et coté calandre (c'est-à-dire à l'extérieur du(des) tube(s)), tout en permettant avantageusement de limiter les pertes de charges à l'intérieur du(des) tube(s) et donc d'éviter l'impact négatif de la baisse de pression sur les performances de la réaction de déshydrogénation, notamment d'éviter une chute de la conversion de l'éthanol.

La taille du réacteur multitubulaire de l'étape c) de déshydrogénation, comme le diamètre de la calandre, pourra être adaptée par l'Homme du métier selon les connaissances générales, en fonction en particulier du nombre de tubes, de leur longueur et de leur diamètre.

Les réacteurs multitubulaires notamment industriels, et en particulier les tubes desdits réacteurs, sont classiquement en un matériau inerte vis-à-vis de la réaction, typiquement en acier ou nickel. Le(s) tubes du (ou des) réacteur(s) multitubulaire(s) dans le procédé de déshydrogénation est(sont) de préférence en acier de tout type, préférentiellement en acier allié et de manière préférée en acier inoxydable. Très avantageusement, la calandre du(ou des) réacteur(s) multitubulaire(s), ainsi que toute chicane éventuellement présente dans la calandre, est dans le même matériau que les tubes réactionnels, de préférence en acier de tout type, préférentiellement en acier allié et de manière préférée en acier inoxydable.

Selon l'invention, la charge est introduite sous forme gazeux dans ledit (ou lesdits) tube(s) de chaque réacteur multitubulaire avantageusement en fonctionnement, de préférence à l'une des extrémités du(desdits) tube(s) réactionnel(s) et de manière préférée simultanément dans l'ensemble des tubes réactionnels du réacteur multitubulaire lorsque celui-ci contient une pluralité de tubes. La charge alimente le (ou les) tube(s) réactionnel(s) à une température d'entrée de ladite charge supérieure ou égale à 240°C, de préférence comprise entre 240°C et 350°C, préférentiellement entre 250°C et 300°C, de manière préférée entre 260°C et 290°C, à une pression d'entrée de ladite charge entre 0,1 et 1,0 MPa, de préférence entre 0,2 et 0,5 MPa, préférentiellement entre 0,3 et 0,4 MPa, et à une vitesse spatiale horaire en poids (PPH) de la charge en entrée du réacteur multitubulaire entre 2 et 15 $h^{-1}$, de préférence entre 2 et 10 $h^{-1}$, en particulier entre 3 et 7 $h^{-1}$, par exemple 5 $h^{-1}$. Ainsi, le débit de la charge en entrée du réacteur multitubulaire, qui comprend par exemple entre 4 000 kg et 30 000 kg, de préférence entre 14 000 kg et 17 000 kg, de catalyseur de déshydrogénation, peut varier entre 20 000 et 150 000 kg/h, préférentiellement entre 50 000 et 100 000 kg/h, de manière préférée entre 70 000 et 85 000 kg/h. Selon l'invention, la vitesse spatiale horaire en poids, encore appelée vitesse pondérale horaire (PPH, poids par poids horaire), peut être définie comme le rapport du débit massique de la charge totale entrant dans le réacteur multitubulaire sur la masse de catalyseur de déshydrogénation compris dans l'ensemble des tubes réactionnels dudit réacteur multitubulaire.

La température d'entrée de ladite charge dans le (ou les) réacteur(s) multitubulaire(s) peut avantageusement être graduellement augmentée, tout en restant avantageusement dans la gamme de températures d'entrée notée ci-dessus, pour compenser au moins en partie la désactivation du catalyseur de déshydrogénation.

L'étape de déshydrogénation du procédé selon l'invention peut éventuellement mettre en œuvre une section de chauffage de la charge, en amont de la section réactionnelle. Le chauffage de la charge dans l'éventuelle section de chauffage peut se faire par toute méthode connue de l'Homme du métier, par exemple par échange de chaleur avec un fluide qui peut être de manière très particulière le fluide caloporteur circulant dans la calandre.

Le flux de la charge peut être en mode ascendant ou descendant, de préférence descendant, dans chaque tube.

Selon l'invention, un fluide caloporteur circule dans la calandre, du (ou des) réacteur(s) multitubulaire(s) avantageusement en fonctionnement, en particulier entre lesdits tubes réactionnels, avantageusement à co-courant ou à contre-courant, de manière préférée à co-courant, du flux circulant à l'intérieur des tubes réactionnels. Le fluide caloporteur est introduit dans la calandre du (ou des) réacteur(s) multitubulaire(s) sous forme gazeux et est, en sortie de calandre, au moins en partie sous forme liquide (c'est-à-dire sous forme liquide ou en mélange gaz-liquide). En d'autres termes, le fluide caloporteur est avantageusement introduit dans la calandre sous forme de vapeur saturante (c'est-à-dire en phase vapeur au point de bulle) et se condense en partie (ou au moins en partie) au contact des tubes à l'intérieur desquels a lieu la réaction de déshydrogénation qui est une réaction endothermique. L'apport de chaleur nécessaire pour maintenir la température dans le(s) tube(s) dans une gamme de températures compatible avec la réaction de déshydrogénation, en particulier au moins égale à 230°C, de préférence supérieure ou égale à 240°C, de manière très préférée supérieure ou égale à 250°C, est donc assuré très avantageusement par l'enthalpie de changement de phase, en particulier de condensation, du fluide caloporteur utilisé.

Le fluide caloporteur est choisi de sorte à être thermiquement stable dans les conditions de fonctionnement décrites ci-dessus. Le choix du fluide caloporteur peut être également guidé par d'autres contraintes : de manière préférée, le fluide caloporteur est inerte vis-à-vis des réactifs et des produits de la réaction de déshydrogénation ; de préférence, le fluide caloporteur n'induit pas de corrosion des équipements, comme le réacteur multitubulaire ou les conduits. Très avantageusement, le fluide caloporteur présente un point d'ébullition unique à une pression donnée. Il est en particulier choisi avantageusement de sorte qu'il présente un point d'ébullition ou une gamme de températures de vapeur saturante (qui est fonction des pressions de vapeur) compatible avec la réaction de déshydrogénation et/ou tel que son enthalpie de changement de phases de l'état gazeux à l'état liquide couvre le besoin énergétique de la réaction de déshydrogénation. De préférence, le fluide caloporteur est une huile, comprenant de manière préférée un mélange eutectique de composés organiques, de préférence de deux composés organiques ayant très avantageusement des points d'ébullition proches, de préférence avec des pressions de vapeur saturante telles que l'écart entre les pressions de vapeur saturante des composés organiques de l'huile, à une température donnée, est inférieur ou égal à 50 Pa, de préférence inférieur ou égal à 20 Pa, de manière préférée inférieur ou égal à 10 Pa. Plus particulièrement, le fluide caloporteur comprend, de préférence consiste en, un mélange de biphényle et d'oxyde de diphényle. Par exemple, le fluide caloporteur est l'huile commercialisé par Dow sous le nom DOWTHERM™ A.

Avantageusement, le fluide caloporteur est introduit dans la calandre du réacteur multitubulaire avantageusement en fonctionnement, à une température d'entrée du fluide caloporteur supérieure ou égale à 260°C, de préférence supérieure ou égale à 270°C, de manière préférée supérieure ou égale à 290°C, et inférieure ou égale à 400°C, de préférence inférieure ou égale à 380°C, et à une pression d'entrée du fluide caloporteur supérieure ou égale à 0,10 MPa, de préférence supérieure ou égale à 0,13 MPa, de manière préférée supérieure ou égale à 0,20 MPa, et inférieure ou égal à 1,10 MPa, de préférence inférieure ou égale 1,06 MPa et de manière préférée inférieure ou égale à 0,85 MPa. Plus particulièrement, le fluide caloporteur est introduit dans la calandre, avantageusement sous forme de vapeur saturante, de préférence à une température supérieure ou égale à 260°C et une pression supérieure ou égale à 0,10 MPa, préférentiellement une température supérieure ou égale à 270°C et une pression supérieure ou égale à 0,13 MPa, de manière préférée à une température supérieure ou égale à 290°C et une pression supérieure ou égale à 0,20 MPa, et de préférence à une température inférieure ou égale à 400°C et une pression inférieure ou égal à 1,10 MPa, de manière préférée à une température inférieure ou égale à 380°C et une pression inférieure ou égale à 0,85 MPa.

La température et/ou pression d'entrée du fluide caloporteur dans la calandre peu(ven)t être graduellement augmentées, avantageusement dans les gammes de température et pression d'entrée notées ci-dessus, pour compenser au moins en partie la désactivation du catalyseur de déshydrogénation.

Le débit massique dudit fluide caloporteur dans la calandre est avantageusement ajusté de sorte que le ratio du débit massique dudit fluide caloporteur dans la calandre par rapport au débit massique de la charge introduite dans le(ou les) tubes est supérieur ou égal à 1,0, de préférence supérieur ou égale à 1,5, et avantageusement inférieur ou égal à 10,0, de préférence inférieur ou égal à 5,0, de manière préférée inférieur ou égal à 2,0.

Dans ces conditions, et comme le coefficient de transfert coté calandre (c'est-à-dire coté condensation) est très supérieur au coefficient de transfert au sein des tubes, la température reste avantageusement constante le long de chacun des tubes du réacteur et égale à la température de condensation du fluide caloporteur. La température est ainsi homogène pour tous les tubes et très proche de la température de la calandre, ce qui est intéressant d'un point de vue conception du réacteur puisque, la température étant homogène dans tout le réacteur, la dilatation du matériau du réacteur, sera la même entre les tubes et entre les tubes et la calandre lors du fonctionnement, conduisant à une réduction du coût de l'équipement.

Avantageusement, le procédé de déshydrogénation peut comprendre une étape de conditionnement du fluide caloporteur comprenant une phase de récupération du fluide caloporteur en sortie de calandre du réacteur multitubulaire de l'étape de déshydrogénation, suivie d'une phase de compression et/ou chauffage du fluide caloporteur pour obtenir un fluide caloporteur sous forme gazeux à la température et la pression d'entrée du fluide caloporteur dans la calandre de l'étape de déshydrogénation.

Dans un tel réacteur et avec les conditions opératoires particulières du procédé selon l'invention, notamment en utilisant l'enthalpie de condensation du fluide caloporteur introduit dans la calandre à des température et pressions spécifiques et à un débit ajusté par rapport à celui de la charge dans les tubes réactionnels, la réaction de déshydrogénation de l'éthanol en acétaldéhyde se déroule avantageusement dans des conditions isothermes ou pseudo-isothermes, c'est-à-dire telle que la température du milieu réactionnel en sortie de réacteur (c'est-à-dire de l'effluent de déshydrogénation en sortie de réacteur) est similaire à la température en entrée de la charge ou présente un écart de moins de 30°C, de préférence de moins de 15°C, par rapport à la température de la charge en entrée de réacteur. Avantageusement, dans de telles conditions, l'effluent de déshydrogénation obtenu à l'issue du réacteur multitubulaire avantageusement en fonction présente une température de préférence supérieure ou égale à 230°C, préférentiellement supérieure ou égale à 240°C, de manière préférée supérieure ou égale à 250°C et de manière préférée supérieure ou égale à 260°C, et de préférence inférieure ou égale à 350°C, préférentiellement inférieure ou égale à 300°C, de manière préférée inférieure ou égale à 290°C, et une pression en sortie de réacteur comprise par exemple entre 0,1 et 0,5 MPa et de préférence entre 0,2 et 0,4 MPa.

Les conditions spécifiques du procédé selon l'invention permettent d'atteindre ainsi les performances souhaitées. En particulier, l'utilisation d'un réacteur-échangeur dans les conditions opératoires spécifiques de l'invention permet très avantageusement d'obtenir des taux de conversion de l'éthanol d'au moins 25%, de préférence d'au moins 30%, voire une conversion de l'éthanol de 35%, et une sélectivité en acétaldéhyde élevée, en particulier au moins 90% poids. Et ces performances sont atteintes sans ajout d'un diluant thermique qui pourrait avoir des conséquences néfastes sur l'activité du catalyseur de déshydrogénation et sans multiplier le nombre de lits catalytiques et/ou de réacteurs comme dans d'un procédé comprenant un enchainement de réacteurs adiabatiques, permettant ainsi de limiter les coûts d'investissement et de fonctionnement. La méthode d'apport de chaleur proposée par l'invention permet également d'adapter aisément les conditions opératoires à l'évolution et la désactivation éventuelle du catalyseur.

Selon l'invention, la conversion de la charge éthanol est définie, en pourcentage massique, par la formule suivante :

$$1 - (\text{masse horaire d'éthanol en sortie/masse horaire d'éthanol en entrée}) \times 100.$$

La masse horaire d'éthanol en entrée ou en sortie correspond au débit massique en entrée ou en sortie du réacteur multitubulaire, et peut être déterminée de manière classique par exemple par chromatographie en phase gazeuse.

Lors de l'étape de déshydrogénation, la transformation de la charge peut s'accompagner d'une désactivation du catalyseur de déshydrogénation, par exemple par cokage, par adsorption de composés inhibiteurs et/ou par frittage. Le catalyseur de déshydrogénation peut donc subir avantageusement périodiquement une régénération ou un remplacement. Ainsi, dans un mode de réalisation particulier de l'invention, le procédé comprend une étape de régénération-remplacement. Dans ce mode de de réalisation particulier, la section réactionnelle comprend de préférence au moins deux réacteurs multitubulaires. De préférence, les réacteurs multitubulaires sont utilisés dans un mode alterné, aussi appelé en mode swing, afin d'alterner les phases de réaction (ou fonctionnement) et les phases de régénération et/ou de remplacement dudit catalyseur de déshydrogénation. L'objectif de la régénération est de brûler les dépôts organiques ainsi que les espèces contenant de l'azote et du soufre, contenues à la surface et au sein dudit catalyseur de déshydrogénation. Le remplacement permet de remplacer le catalyseur usé, c'est-à-dire qui a été utilisé lors d'au moins une étape de déshydrogénation, par du catalyseur de déshydrogénation frais, c'est-à-dire n'ayant pas encore servi. La régénération du catalyseur de déshydrogénation peut être avantageusement réalisée par oxydation du coke et des composés inhibiteurs sous flux d'air ou sous un mélange air/azote, par exemple en utilisant une recirculation de l'air de combustion avec ou sans eau afin de diluer l'oxygène et maîtriser l'exotherme de régénération. Dans ce cas, la teneur en oxygène est avantageusement ajustée en entrée du réacteur par un appoint d'air. La régénération a lieu de préférence à pression comprise entre la pression atmosphérique et la pression de réaction.

Selon un mode de réalisation très particulier de l'invention, l'étape de régénération-remplacement comprend :

- le remplacement du catalyseur de déshydrogénation, notamment le remplacement du catalyseur usé par du catalyseur frais, de préférence comprenant au moins l'élément cuivre sur un support inorganique préférentiellement la silice, ou

- la régénération du catalyseur de déshydrogénation comprenant de préférence au moins trois phases, avec au moins une première phase d'un balayage à l'azote à une température de préférence comprise entre 200 et 350°C, de préférence entre 250 et 300°C, au moins une deuxième phase d'un balayage par un gaz comprenant de l'oxygène, de préférence comprenant de l'azote et de l'oxygène, à une température comprise entre 300 et 650°C, de préférence entre 350°C et 600°C, avantageusement jusqu'à ce qu'il n'y a plus de consommation d'oxygène, signe d'une combustion totale du coke, et au moins une troisième phase d'un balayage à l'azote à une température de préférence comprise entre 200 et 350°C. La régénération peut éventuellement comprendre, en outre, une phase de redispersion de la phase active comprenant de préférence au moins l'élément cuivre et éventuellement l'élément chrome, sur le support inorganique, de préférence la silice.

Très avantageusement, l'effluent de déshydrogénation obtenu à l'issue de la section réactionnelle de l'étape de déshydrogénation, qui comprend au moins de l'acétaldéhyde, de l'hydrogène, éventuellement de l'eau, et de l'éthanol non converti, peut être envoyé dans une section de séparation pour éventuellement séparer au moins en partie l'hydrogène généré lors de la réaction de déshydrogénation.

L'effluent de déshydrogénation obtenu à l'issue de la section réactionnelle, ou l'effluent obtenu à l'issue de la section de séparation, peut également subir, soit directement, soit indirectement, un traitement de manière à séparer un flux comprenant l'éthanol non converti, ledit flux d'éthanol non converti pouvant alors être recyclé vers la section réactionnelle pour alimenter le(ou les) réacteur(s) multitubulaire(s) éventuellement en mélange avec la charge.

Avantageusement, le procédé de déshydrogénation selon l'invention peut être intégré comme étape réactionnelle dans un procédé plus global de conversion d'éthanol. En particulier, le procédé de déshydrogénation selon l'invention peut être intégré dans un procédé de production de butadiène à partir d'éthanol, comme première étape réactionnelle de conversion de l'éthanol en acétaldéhyde et est avantageusement suivi d'une deuxième étape réactionnelle de conversion d'un mélange éthanol-acétaldéhyde en butadiène. Un tel procédé de production de butadiène à partir d'éthanol, en deux étapes réactionnelles, peut par exemple être le procédé décrit dans le brevet FR 3 026 100. Plus particulièrement, l'étape A) du procédé décrit dans le brevet FR 3 026 100 est remplacée par le procédé de déshydrogénation décrit plus haut dans cette description, et les étapes B), C1), D1), D2), D3), E1), E2), et les étapes optionnelles C2), D2bis), F) du procédé décrit dans le brevet FR 3 026 100 restant identiques.

Ainsi, la présente invention concerne en outre un procédé de production de butadiène à partir d'une charge éthanol comprenant au moins 80% poids d'éthanol, comprenant au moins :

A) une étape de conversion de l'éthanol en acétaldéhyde mettant en œuvre le procédé de déshydrogénation de l'éthanol décrit plus haut dans lequel ladite charge qui alimente les tubes du réacteur multitubulaire est au moins en partie une fraction d'un effluent riche en éthanol, avantageusement issu de l'étape E1), pour produire un effluent de déshydrogénation, et éventuellement une section de séparation pour traiter l'effluent de déshydrogénation et séparer au moins un effluent hydrogène sous forme gazeuse et un effluent éthanol/acétaldéhyde sous forme liquide ;

B) une étape de conversion en butadiène comprenant au moins une section réactionnelle B alimentée au moins par une fraction ou la totalité dudit effluent de déshydrogénation issu de l'étape A), ou de l'éventuel effluent éthanol/acétaldéhyde issu de l'éventuelle section de séparation de l'étape A), éventuellement par un effluent liquide riche en éthanol avantageusement issu de l'éventuelle étape C1), par une fraction ou la totalité d'un effluent riche en acétaldéhyde avantageusement issu de l'étape E1), opérée en présence d'un catalyseur, de préférence comprenant l'élément tantale et un support inorganique comprenant de préférence de la silice, à une température comprise entre 300°C et 400°C, de préférence entre 320°C et 370°C, et à une pression comprise entre 0,1 et 1,0 MPa, de préférence entre 0,1 et 0,5 MPa, de manière préférée entre 0,1 et 0,3 MPa, les débits d'alimentation étant réglés de telle sorte que le rapport molaire de l'éthanol par rapport à l'acétaldéhyde en entrée de ladite section réactionnelle est compris entre 1 et 5, de préférence entre 1 et 3,5, de manière préférée entre 2 et 3 et de manière très préférée entre 2,4 et 2,7, et une section de séparation pour traiter l'effluent de ladite section réactionnelle B et séparer au moins un effluent gazeux et un effluent liquide;

C1) éventuellement une étape de traitement de l'hydrogène comprenant au moins une section de compression comprimant ledit effluent hydrogène issu de l'étape A) à une pression comprise entre 0,1 et 1,0 MPa, avantageusement entre 0,1 et 0,7 MPa, de manière préférée entre 0,4 et 0,68 MPa, et une section de lavage gaz-liquide alimentée à une température comprise entre 15°C et -30°C, de préférence entre 0°C et -15°C, par une fraction dudit effluent riche en éthanol avantageusement issu de l'étape E1) et par une fraction dudit effluent éthanol/acétaldéhyde issu de l'étape A), et alimentée à une température comprise entre 25°C et 60°C, préférentiellement entre 30°C et 40°C, par ledit effluent hydrogène comprimé, et produisant au moins un effluent liquide riche en éthanol et un effluent hydrogène purifié ;

D1) une étape d'extraction du butadiène comprenant au moins :

(i) une section de compression comprimant ledit effluent gazeux issu de l'étape B) à une pression comprise entre 0,1 et 1,0 MPa, de préférence entre 0,1 et 0,7 MPa, de manière préférée entre 0,2 et 0,5 MPa, éventuellement ledit effluent gazeux issu de l'étape B) comprimé étant ensuite refroidi à une température entre 25°C et 60°C, préférentiellement entre 30°C et 40°C,

(ii) une section de lavage gaz-liquide comprenant une colonne de lavage alimentée en tête à une température comprise entre 20 et -20°C, préférentiellement entre 15°C et 5°C, par un flux éthanol constitué de la charge éthanol du procédé et éventuellement d'une fraction de l'effluent riche en éthanol avantageusement issu de l'étape E1), et en fond par ledit effluent gazeux issu de l'étape B) comprimé dans la section i) et éventuellement refroidi, produisant au moins un effluent liquide de lavage et un effluent sous-produits gazeux, et

(iii) une section de distillation opérée à une pression comprise entre 0,1 et 1 MPa, de manière préférée entre 0,2 et 0,5 MPa, alimentée au moins par l'effluent liquide issu de ladite étape B) et par l'effluent liquide de ladite section de lavage gaz-liquide, produisant au moins un effluent butadiène brut et un effluent éthanol/acétaldéhyde/eau ;

D2) une étape de première purification du butadiène comprenant au moins une section de lavage gaz-liquide alimentée en fond par l'effluent butadiène brut issu de D1) et en tête par un flux d'eau pouvant être un flux d'eau d'origine externe audit procédé de production de butadiène et/ou une fraction de l'effluent eau issu avantageusement de l'étape E1), ledit flux d'eau étant de préférence refroidi préalablement à la section de lavage gaz-liquide à une température inférieure à 25°C, préférentiellement inférieure à 20°C, ladite section de lavage gaz-liquide étant avantageusement opérée à une pression comprise entre 0,1 et 1 MPa, ladite section de lavage gaz-liquide produisant en tête un effluent butadiène pré-purifié et en fond un effluent eau usée ;

D3) une étape ultérieure de purification du butadiène, alimentée au moins par ledit effluent butadiène pré-purifié issu de ladite étape D2), et produisant au moins un effluent butadiène purifié, ladite étape ultérieure de purification mettant avantageusement en œuvre une section de séchage dudit effluent butadiène pré-purifié issu de ladite étape D2) de préférence en présence d'au moins un adsorbant, puis au moins une section de distillation cryogénique ou au moins une section de distillation et distillation extractive ;

E2) une étape d'élimination des impuretés et des huiles brunes, alimentée au moins par l'effluent éthanol/acétaldéhyde/eau issu de l'étape D1), et par au moins une fraction d'un effluent riche en eau avantageusement issu de l'étape E1), et produisant au moins un raffinat eau / éthanol / acétaldéhyde, un effluent huiles brunes légères et un effluent huiles brunes lourdes,

ladite étape d'élimination des impuretés et des huiles brunes mettant de préférence en œuvre au moins :

E2i) une section de lavage/contre-lavage opérée à une pression entre 0,1 et 0,5 MPa, préférentiellement entre 0,2 et 0,4 MPa, et alimentée l'effluent éthanol/acétaldéhyde/eau issu de l'étape D1) et préférentiellement en fond par un effluent hydrocarbures et en tête par au moins une fraction de de l'effluent riche en eau avantageusement issu de l'étape E1), lesdits effluents hydrocarbures et riche en eau étant de préférence à une température entre 10 et 70°C, préférentiellement entre 45 et 55°C, et produisant ledit raffinat eau / éthanol / acétaldéhyde et un extrait hydrocarbures,

E2ii) une section de distillation des huiles brunes légères alimentée par l'extrait hydrocarbures et produisant ledit effluent huiles brunes légères et un résidu hydrocarbures, et

E2iii) une section de distillation des huiles brunes lourdes alimentée par le résidu hydrocarbures et produisant ledit effluent huiles brunes lourdes et un distillat hydrocarbures, qui compose avantageusement au moins en partie l'effluent hydrocarbures de la section de lavage/contre-lavage ;

E1) une étape traitement des effluents alimentée au moins par un raffinat eau/éthanol/acétaldéhyde avantageusement issu de l'étape E2), mettant de préférence en œuvre au moins deux sections de distillation, en particulier au moins une section de distillation de l'acétaldéhyde et au moins une section de distillation de l'eau et de l'éthanol, et ladite étape traitement des effluents produisant au moins un effluent riche en éthanol, de préférence comprenant au moins 80% poids d'éthanol, un effluent riche en acétaldéhyde, de préférence comprenant au moins 80% poids d'acétaldéhyde, et un effluent riche en eau, de préférence comprenant au moins 80% poids d'eau.

Dans ce mode de réalisation particulier de l'invention, l'effluent riche en éthanol obtenu à l'étape E1 et recyclé comme charge de l'étape A) au cours de laquelle est réalisée la déshydrogénation de l'éthanol, peut comprendre classiquement jusqu'à 18% poids d'eau.

Ainsi, le procédé de déshydrogénation selon l'invention apparait particulièrement avantageux dans ce procédé de production de butadiène dans la mesure où l'effluent riche en éthanol, alimentant la section réactionnelle de déshydrogénation, comprenant classiquement jusqu'à 18% poids d'eau, ne permet pas d'envisager un ajout de vapeur d'eau comme diluant thermique dans la charge de l'étape de déshydrogénation sans désactivation précoce du catalyseur de déshydrogénation dont les performances sont réduites généralement lorsque les charges comprennent 20% poids ou plus d'eau.

Les exemples suivants illustrent l'invention sans en limiter la portée.

**Exemples**

**Exemple 1 : conforme à l'invention**

L'exemple 1 illustre un procédé de déshydrogénation selon l'invention.

La charge à traiter comprend 82% poids d'éthanol et 18% poids d'eau.

La réaction de déshydrogénation est mis en œuvre dans un réacteur multitubulaire en acier allié dont les tubes comprennent un lit fixe de catalyseur Octolyst® 2001 commercialisé par Evonik. La charge est introduite dans les tubes sous forme gazeux, de manière simultanée. Le fluide caloporteur utilisé est l'huile Dowtherm™ A de Dow, et est introduit dans la calandre sous forme gazeux, en particulier sous forme de vapeur saturante.

Le Tableau 1 résume l'ensemble des paramètres du réacteur et des conditions opératoires utilisés.

Tableau 1

| Paramètres (unité) | Valeur |
|---|---|
| Nombre de tubes (-) | 5312 |
| Hauteur des tubes (m) | 2,5 |
| Diamètre interne des tubes (mm) | 45,2 |
| Epaisseur de la paroi des tubes (mm) | 2,77 |
| Masse de catalyseur (kg) | 15 670 |
| Diamètre moyen équivalent des particules de catalyseur (mm) | 4 |
| Température d'entrée de la charge (°C) | 270 |
| Pression d'entrée de la charge (MPa) | 0,35 |
| Débit de la charge (kg/h) | 78 346 |
| PPH ($h^{-1}$) charge totale | 5 |
| Température de l'huile en entrée de la calandre (°C) | 290 |
| Pression de l'huile en entrée de la calandre (MPa) | 0,198 |
| Débit de l'huile en entrée de calandre (kg/h) | 120 000 |
| Ratio des débits pondéraux entre l'huile et la charge | Environ 1,53 |

L'effluent de déshydrogénation est récupéré en sortie de réacteur à un débit de 78 346 kg/h, une température d'environ 277°C et une pression d'environ 0,29 MPa (soit une perte de charge d'environ 0,6 bar, c'est-à-dire environ 0,06 MPa). En sortie de calandre, un mélange gaz-liquide d'huile Dowtherm™ A, à 290°C, est récupéré.

L'effluent de déshydrogénation obtenu est analysé par chromatographie en phase gaz. Il présente la composition suivante :

57% poids d'éthanol
22% poids d'acétaldéhyde,
18% poids d'eau,
2% poids de composés autres, et en particulier : l'acétate d'éthyle, de l'acide acétique et du butanol, environ 1% poids d'hydrogène.

Les performances obtenues du procédé sont satisfaisantes puisque le procédé permet d'atteindre une conversion de 35% poids de l'éthanol avec une sélectivité en acétaldéhyde de 92%.

**Exemple 2 : non conforme à l'invention**

L'exemple 2 illustre un procédé mettant en œuvre la réaction de déshydrogénation en réacteurs adiabatiques.

La même charge que celle de l'exemple 1 est traitée par le procédé de l'exemple 2 : elle comprend 18% poids d'eau et 82% poids d'éthanol.

Le même catalyseur Octolyst® 2001 d'Evonik est utilisé comme catalyseur de déshydrogénation.

La réaction de déshydrogénation est mise en œuvre dans une succession de 11 réacteurs adiabatiques axiaux en série comprenant chacun un lit fixe de catalyseur de déshydrogénation (Octolyst® 2001) et entre lesquels sont insérés des échangeurs de chaleur pour réchauffer le flux liquide entre chaque lit. L'unité réactionnelle comprend donc 11 réacteurs adiabatiques en série et 10 échangeurs de chaleur. La charge qui comprend 82% poids d'éthanol et 18% poids d'eau, est introduite dans le premier réacteur à une température d'entrée de 275°C, à une pression d'entrée de 0,57 MPa et à un débit de 78 346 kg/h, correspondant à une pph de 2 h$^{-1}$ par rapport à l'éthanol.

Le Tableau 2 présente les paramètres des réacteurs adiabatiques à lits fixes axiaux, les conditions opératoires et les taux de conversion de l'éthanol obtenus. La pression en sortie du onzième réacteur est de 0,25 MPa.

Tableau 2

| N° Réacteur | 1 | 2 | 3 | 4 | 5 | 6 | 7 | 8 | 9 | 10 | 11 |
|---|---|---|---|---|---|---|---|---|---|---|---|
| Volume du lit catalytique (m$^3$) | 1,7 | 3,3 | 4,2 | 4,2 | 4,2 | 4,2 | 4,2 | 4,2 | 4,2 | 4,2 | 4,2 |
| Volume cumulé (m$^3$) | 1,7 | 5,0 | 9,2 | 13,4 | 17,6 | 21,8 | 26,0 | 30,2 | 34,4 | 38,6 | 42,8 |
| $T_{entrée}$ (°C) | 275 | 275 | 275 | 275 | 275 | 275 | 275 | 275 | 275 | 275 | 275 |
| $T_{sortie}$ (°C) | 233 | 244 | 253 | 259 | 262 | 265 | 267 | 267 | 267 | 267 | 267 |
| Conversion (%) | 8 | 14 | 18 | 21 | 24 | 26 | 28 | 29 | 31 | 33 | 34 |

En sortie de l'unité réactionnelle, une conversion en éthanol de 34% est atteinte et la sélectivité en acétaldéhyde est de 92%.

**Exemple 3 : non conforme à l'invention**

L'exemple 3 illustre un procédé mettant en œuvre la réaction de déshydrogénation en réacteurs adiabatiques en présence d'un diluant thermique.

Le même catalyseur Octolyst® 2001 d'Evonik que celui utilisé dans les procédés décrits en Exemple 1 et Exemple 2 est utilisé comme catalyseur de déshydrogénation.

La charge qui est traitée par le procédé de l'exemple 3 est quant à elle diluée par de la vapeur d'eau jusqu'à une dilution de 60% poids d'eau pour 40% poids d'éthanol. Cette charge est introduite dans un enchainement de réacteurs adiabatiques radiaux comprenant chacun un lit fixe de catalyseur de déshydrogénation et entre lesquels sont insérés des échangeurs de chaleur pour réchauffer le flux liquide entre chaque lit. La charge qui comprend 40% poids d'éthanol et 60% poids d'eau, est introduite dans le premier réacteur à une température d'entrée de 275°C, à une pression d'entrée de 0,37 MPa et à un débit de 78 346 kg/h.

Le Tableau 3 présente les paramètres des réacteurs adiabatiques à lits fixes radiaux, les conditions opératoires et les taux de conversion de l'éthanol obtenus. La pression en sortie du réacteur n°4 est 0,20 MPa.

Tableau 3

| N° Réacteur | 1 | 2 | 3 | 4 |
|---|---|---|---|---|
| Volume du lit catalytique (m$^3$) | 1,7 | 3,3 | 4,2 | 4,2 |
| Volume cumulé (m$^3$) | 1,7 | 5,0 | 9,2 | 13,4 |
| $T_{entrée}$ (°C) | 275 | 275 | 275 | 275 |
| $T_{sortie}$ (°C) | 225 | 254 | 255 | 260 |
| Conversion (%) | 16 | 23 | 30 | 35 |

Une dilution de l'éthanol de 60% poids par de la vapeur d'eau permet d'atteindre une conversion de l'éthanol de 35% après seulement 4 réacteurs adiabatiques.

Cependant, dans les conditions du procédé de l'Exemple 3 et en particulier avec une charge éthanol diluée à 60% poids d'eau, une désactivation du catalyseur de déshydrogénation plus rapide que dans le procédé de l'Exemple 2 est observée.

**Revendications**

1. Procédé de déshydrogénation de l'éthanol en acétaldéhyde, comprenant une étape de déshydrogénation d'une charge comprenant de l'éthanol, ladite étape de déshydrogénation mettant en œuvre une section réactionnelle comprenant au moins un réacteur multitubulaire qui comprend un ou une pluralité de tubes et une calandre,

   ledit (ou lesdits) tube(s) comprenant chacun au moins un lit fixe d'au moins un catalyseur de déshydrogénation, ladite charge alimentant ledit (ou lesdits) tube(s) sous forme gazeux, à une température d'entrée de ladite charge supérieure ou égale à 240°C, à une pression d'entrée de ladite charge entre 0,1 et 1,0 MPa, et à une vitesse spatiale horaire en poids (PPH) de la charge en entrée entre 2 et 15 h$^{-1}$,
   un fluide caloporteur circulant dans ladite calandre de sorte que ledit fluide caloporteur est introduit dans ladite calandre sous forme gazeux et est, en sortie de calandre, au moins en partie sous forme liquide, ledit fluide caloporteur étant introduit dans la calandre à un débit tel que le ratio du débit pondéral dudit fluide caloporteur en entrée de calandre par rapport au débit pondéral de ladite charge en entrée du (ou des) tubes est supérieur ou égal à 1,0,
   ledit fluide caloporteur étant introduit dans la calandre à une température d'entrée du fluide caloporteur supérieure ou égale à 260°C, et inférieure ou égale à 400°C, et à une pression d'entrée du fluide caloporteur supérieure ou égale à 0,10 MPa, et inférieure ou égal à 1,10 MPa ;
   ledit procédé produisant un effluent de déshydrogénation comprenant au moins de l'acétaldéhyde, de l'hydrogène et de l'éthanol non converti.

2. Procédé selon la revendication 1, dans lequel le fluide caloporteur est une huile, comprenant un mélange eutectique de composés organiques ayant des pressions de vapeur saturante telles que l'écart entre les pressions de vapeur saturante des composés organiques de l'huile à une température donnée est inférieur ou égal à 50 Pa, de préférence inférieur ou égal à 20 Pa, de manière préférée inférieur ou égal à 10 Pa.

3. Procédé selon la revendication 1 ou 2, dans lequel la charge comprend au moins 50% poids d'éthanol, préférentiellement au moins 70% poids d'éthanol, de manière préférée au moins 80% poids d'éthanol, et éventuellement de l'eau, de préférence à une teneur inférieure à 50% poids, préférentiellement inférieure à 30% poids, de manière préférée inférieure à 20% poids.

4. Procédé selon l'une des revendications précédentes, dans lequel ledit au moins un réacteur multitubulaire comprend une pluralité de tubes, de préférence au moins 100 tubes, préférentiellement au moins 1 000 tubes et de manière préférée au moins 2 000 tubes, et de préférence moins de 20 000 tubes, de manière préférée moins de 10 000 tubes, par exemple entre 5 000 et 6 000 tubes.

5. Procédé selon l'une des revendications précédentes, dans lequel le ou les tube(s) du réacteur multitubulaire a(ont) une longueur comprise entre 1 et 6 m, de manière préférée entre 2 et 3 m.

6. Procédé selon l'une des revendications précédentes, dans lequel le ou les tube(s) du réacteur multitubulaire présente(nt) un diamètre interne entre 30,0 et 60,0 mm, préférentiellement entre 40,0 et 50,0 mm, et une épaisseur de paroi de tube de préférence entre 1,5 et 5,0 mm, préférentiellement entre 2,0 et 4,0 mm et de manière préférée entre 2,2 et 3,2 mm.

7. Procédé selon l'une des revendications précédentes, dans lequel le catalyseur de déshydrogénation comprend au moins l'élément cuivre sur un support inorganique de manière préférée la silice.

8. Procédé selon l'une des revendications précédentes, dans lequel le catalyseur de déshydrogénation est sous forme de particules de diamètre moyen équivalent entre 0,5 et 10,0 mm, de préférence entre 1,0 et 5,0 mm.

9. Procédé selon l'une des revendications précédentes, dans lequel la température d'entrée de la charge dans le (ou les) tubes est entre 240°C et 350°C, préférentiellement entre 250°C et 300°C, de manière préférée entre 260°C et 290°C.

10. Procédé selon l'une des revendications précédentes, dans lequel la pression d'entrée de la charge dans le (ou les) tubes est entre 0,2 et 0,5 MPa, préférentiellement entre 0,3 et 0,4 MPa.

11. Procédé selon l'une des revendications précédentes, dans lequel la vitesse spatiale horaire en poids (PPH) de la charge en entrée du réacteur est entre 2 et 10 h$^{-1}$,

12. Procédé selon l'une des revendications précédentes, dans lequel le fluide caloporteur est introduit dans la calandre du réacteur multitubulaire à un débit tel que le ratio du débit pondéral dudit fluide caloporteur en entrée de calandre par rapport au débit pondéral de ladite charge en entrée du (ou des) tubes est supérieure ou égale à 1,5, et avantageusement inférieur ou égal à 10,0, de préférence inférieur ou égal à 5,0, de manière préférée inférieur ou égal à 2,0,

13. Procédé selon l'une des revendications précédentes, dans lequel le fluide caloporteur est introduit dans la calandre du réacteur multitubulaire à une température d'entrée supérieure ou égale à 260°C, de préférence supérieure ou égale à 270°C, de manière préférée supérieure ou égale à 290°C, et inférieure ou égale à 400°C, de préférence inférieure ou égale à 380°C, et à une pression d'entrée du fluide caloporteur supérieure ou égale à 0,10 MPa, de préférence supérieure ou égale à 0,13 MPa, de manière préférée supérieure ou égale à 0,20 MPa, et inférieure ou égal à 1,10 MPa, de manière préférée inférieure ou égale à 0,85 MPa.

14. Procédé selon l'une des revendications précédentes, comprenant une étape de conditionnement du fluide caloporteur comprenant une sous-étape de récupération du fluide caloporteur liquide en sortie de calandre du réacteur multitubulaire de l'étape de déshydrogénation et une étape de compression et/ou chauffage du fluide caloporteur pour obtenir un fluide caloporteur sous forme gazeux à la température et la pression d'entrée du fluide caloporteur dans la calandre de l'étape de déshydrogénation.

15. Procédé de production de butadiène à partir d'une charge éthanol comprenant au moins 80% poids d'éthanol, comprenant :

A) une étape de conversion de l'éthanol en acétaldéhyde mettant en œuvre le procédé de déshydrogénation de l'éthanol selon l'une quelconque des revendication 1 à 14 dans lequel ladite charge qui alimente les tubes du réacteur multitubulaire est au moins en partie une fraction d'un effluent riche en éthanol, avantageusement issu de l'étape E1), pour produire un effluent de déshydrogénation, et éventuellement une section de séparation pour traiter l'effluent de déshydrogénation et séparer au moins un effluent hydrogène sous forme gazeuse et un effluent éthanol/acétaldéhyde sous forme liquide ;
B) une étape de conversion en butadiène comprenant au moins une section réactionnelle B alimentée au moins par une fraction ou la totalité dudit effluent de déshydrogénation issu de l'étape A), ou de l'éventuel effluent éthanol/acétaldéhyde issu de l'éventuelle section de séparation de l'étape A), éventuellement par un effluent liquide riche en éthanol avantageusement issu de l'étape C1), par une fraction ou la totalité d'un effluent riche en acétaldéhyde avantageusement issu de l'étape E1), opérée en présence d'un catalyseur, de préférence comprenant l'élément tantale et un support inorganique comprenant de préférence de la silice, à une température comprise entre 300 et 400°C, et à une pression comprise entre 0,1 et 1,0 MPa, les débits d'alimentation étant réglés de telle sorte que le rapport molaire de l'éthanol par rapport à l'acétaldéhyde en entrée de ladite section réactionnelle est compris entre 1 et 5, et une section de séparation pour traiter l'effluent de ladite section réactionnelle B et séparer au moins un effluent gazeux et un effluent liquide ;
C1) éventuellement une étape de traitement de l'hydrogène comprenant au moins une section de compression comprimant ledit effluent hydrogène issu de l'étape A) à une pression comprise entre 0,1 et 1,0 MPa, et une section de lavage gaz-liquide alimentée à une température comprise entre 15°C et - 30°C par une fraction dudit effluent riche en éthanol avantageusement issu de l'étape E1) et par une fraction dudit effluent éthanol/acétaldéhyde issu de l'étape A), et alimentée à une température comprise entre 25°C et 60°C par ledit effluent hydrogène comprimé, et produisant au moins un effluent liquide riche en éthanol et un effluent hydrogène purifié ;
D1) une étape d'extraction du butadiène comprenant au moins :

(i) une section de compression comprimant ledit effluent gazeux issu de l'étape B) à une pression comprise entre 0,1 et 1,0 MPa, éventuellement ledit effluent gazeux issu de l'étape B) comprimé étant ensuite refroidi à une température entre 25°C et 60°C,
(ii) une section de lavage gaz-liquide comprenant une colonne de lavage alimentée en tête à une température comprise entre 20 et -20°C par un flux éthanol constitué de la charge éthanol du procédé et éventuellement d'une fraction de l'effluent riche en éthanol avantageusement issu de l'étape E1), et en fond par ledit effluent gazeux issu de l'étape B) comprimé et éventuellement refroidi, produisant au moins un effluent liquide de lavage et un effluent sous-produits gazeux, et

(iii) une section de distillation opérée à une pression comprise entre 0,1 et 1 MPa, alimentée au moins par l'effluent liquide issu de ladite étape B) et par l'effluent liquide de ladite section de lavage gaz-liquide, produisant au moins un effluent butadiène brut et un effluent éthanol/acétaldéhyde/eau ;

D2) une étape de première purification du butadiène comprenant au moins une section de lavage gaz-liquide alimentée en fond par l'effluent butadiène brut issu de D1) et en tête par un flux d'eau qui est un flux d'eau d'origine externe audit procédé de production de butadiène et/ou une fraction de l'effluent eau issu avantageusement de l'étape E1), ladite section de lavage produisant en tête un effluent butadiène pré-purifié et en fond un effluent eau usée ;

D3) une étape ultérieure de purification du butadiène, alimentée au moins par ledit effluent butadiène pré-purifié issu de ladite étape D2), et produisant au moins un effluent butadiène purifié ;

E2) une étape d'élimination des impuretés et des huiles brunes, alimentée au moins par l'effluent éthanol/acétaldéhyde/eau issu de l'étape D1), et par au moins une fraction d'un effluent riche en eau, avantageusement issu de l'étape E1), et produisant au moins un raffinat eau/éthanol/acétaldéhyde, un effluent huiles brunes légères et un effluent huiles brunes lourdes ;

E1) une étape traitement des effluents alimentée au moins par un raffinat eau/éthanol/acétaldéhyde avantageusement issu de l'étape E2), et produisant au moins un effluent riche en éthanol, un effluent riche en acétaldéhyde et un effluent riche en eau

**Patentansprüche**

1. Verfahren zur Dehydrierung von Ethanol zu Acetaldehyd, das einen Schritt zur Dehydrierung eines Einsatzmaterials umfasst, das Ethanol umfasst, wobei bei dem Schritt zur Dehydrierung ein Reaktionsabschnitt verwendet wird, der mindestens einen Rohrbündelreaktor umfasst, der ein oder mehrere Rohre und einen Mantel umfasst,

wobei das Rohr (oder die Rohre) jeweils mindestens ein Festbett aus mindestens einem Dehydrierungskatalysator umfasst (beziehungsweise umfassen),

wobei das Einsatzmaterial bei einer Eintrittstemperatur des Einsatzmaterials von größer gleich 240 °C, bei einem Eintrittsdruck des Einsatzmaterials zwischen 0,1 und 1,0 MPa und bei einer Massenstundenraumgeschwindigkeit (MSG) des Einsatzmaterials am Einlass zwischen 2 und 15 h$^{-1}$ gasförmig in das Rohr (oder die Rohre) geführt wird,

wobei ein Wärmeträgermedium derart in dem Mantel strömt, dass das Wärmeträgermedium gasförmig in den Mantel geleitet wird und am Ausgang des Mantels zumindest teilweise in flüssiger Form vorliegt,

wobei das Wärmeträgermedium mit einem derartigen Volumenstrom in den Mantel geleitet wird, dass das Verhältnis des gewichtsbezogenen Volumenstroms des Wärmeträgermediums am Eintritt des Mantels bezogen auf den gewichtsbezogenen Volumenstrom des Einsatzmaterials am Eintritt des Rohrs (oder der Rohre) größer gleich 1,0 ist,

wobei das Wärmeträgermedium mit einer Eintrittstemperatur des Wärmeträgermediums von größer gleich 260 °C und kleiner gleich 400 °C und mit einem Eintrittsdruck des Wärmeträgermediums von größer gleich 0,10 MPa und kleiner gleich 1,10 MPa in den Mantel geleitet wird;

wobei mit dem Verfahren ein Dehydrierungsaustrag produziert wird, der mindestens Acetaldehyd, Wasserstoff und nicht umgesetztes Ethanol umfasst.

2. Verfahren nach Anspruch 1, wobei das Wärmeträgermedium ein Öl ist, das eine eutektische Mischung aus organischen Verbindungen mit einem derartigen Sättigungsdampfdruck umfasst, dass der Abstand zwischen dem Sättigungsdampfdruck der organischen Verbindungen des Öls bei einer gegebenen Temperatur kleiner gleich 50 Pa, vorzugsweise kleiner gleich 20 Pa, bevorzugterweise kleiner gleich 10 Pa beträgt.

3. Verfahren nach Anspruch 1 oder 2, wobei das Einsatzmaterial mindestens 50 Gewichts-% Ethanol, vorzugsweise mindestens 70 Gewichts-% Ethanol, bevorzugt mindestens 80 Gewichts-% Ethanol und gegebenenfalls Wasser, vorzugsweise bei einem Gehalt von unter 50 Gewichts-%, bevorzugt unter 30 Gewichts-%, bevorzugterweise unter 20 Gewichts-% umfasst.

4. Verfahren nach einem der vorhergehenden Ansprüche, wobei der mindestens eine Rohrbündelreaktor mehreren Rohren umfasst, vorzugsweise mindestens 100 Rohre, bevorzugt mindestens 1000 Rohre und bevorzugterweise mindestens 2000 Rohre, und vorzugsweise weniger als 20.000 Rohre, bevorzugterweise weniger als 10.000 Rohre, beispielsweise zwischen 5000 und 6000 Rohre.

5. Verfahren nach einem der vorhergehenden Ansprüche, wobei das Rohr oder die Rohre des Rohrbündelreaktors eine Länge zwischen 1 und 6 m, bevorzugterweise zwischen 2 und 3 m aufweist bzw. aufweisen.

6. Verfahren nach einem der vorhergehenden Ansprüche, wobei das Rohr oder die Rohre des Rohrbündelreaktors einen Innendurchmesser zwischen 30,0 und 60,0 mm, vorzugsweise zwischen 40,0 und 50,0 mm, und eine Rohrwanddicke bevorzugt zwischen 1,5 und 5,0 mm, vorzugsweise zwischen 2,0 und 4,0 mm und bevorzugterweise zwischen 2,2 und 3,2 mm aufweist bzw. aufweisen.

7. Verfahren nach einem der vorhergehenden Ansprüche, wobei der Dehydrierungskatalysator mindestens das Element Kupfer auf einem anorganischen Träger, bevorzugterweise Siliciumdioxid, umfasst.

8. Verfahren nach einem der vorhergehenden Ansprüche, wobei der Dehydrierungskatalysator in Form von Teilchen mit einem mittleren Äquivalentdurchmesser zwischen 0,5 und 10,0 mm, bevorzugt zwischen 1,0 und 5,0 mm vorliegt.

9. Verfahren nach einem der vorhergehenden Ansprüche, wobei die Eintrittstemperatur des Einsatzmaterials in das Rohr (oder die Rohre) zwischen 240 °C und 350 °C, vorzugsweise zwischen 250 °C und 300 °C, bevorzugterweise zwischen 260 °C und 290 °C beträgt.

10. Verfahren nach einem der vorhergehenden Ansprüche, wobei der Eintrittsdruck des Einsatzmaterials in das Rohr (oder die Rohre) zwischen 0,2 und 0,5 MPa, vorzugsweise zwischen 0,3 und 0,4 MPa beträgt.

11. Verfahren nach einem der vorhergehenden Ansprüche, wobei die Massenstundenraumgeschwindigkeit (MSG) des Einsatzmaterials am Reaktoreintritt zwischen 2 und 10 $h^{-1}$ beträgt.

12. Verfahren nach einem der vorhergehenden Ansprüche, wobei das Wärmeträgermedium mit einem derartigen Volumenstrom in den Mantel des Rohrbündelreaktors geleitet wird, dass das Verhältnis des gewichtsbezogenen Volumenstromes des Wärmeträgermediums am Eintritt des Mantels bezogen auf den gewichtsbezogenen Volumenstrom des Einsatzmaterials am Eintritt des Rohrs (oder der Rohre) größer gleich 1,5 und vorteilhafterweise kleiner gleich 10,0, bevorzugt kleiner gleich 5,0, bevorzugterweise kleiner gleich 2,0 ist.

13. Verfahren nach einem der vorhergehenden Ansprüche, wobei das Wärmeträgermedium mit einer Eintrittstemperatur von größer gleich 260 °C, bevorzugt größer gleich 270 °C, bevorzugterweise größer gleich 290 °C, und kleiner gleich 400 °C, bevorzugt kleiner gleich 380 °C, und bei einem Eintrittsdruck des Wärmeträgermediums von größer gleich 0,10 MPa, bevorzugt größer gleich 0,13 MPa, bevorzugterweise größer gleich 0,20 MPa, und kleiner gleich 1,10 MPa, bevorzugterweise kleiner gleich 0,85 MPa in den Mantel des Rohrbündelreaktors geleitet wird.

14. Verfahren nach einem der vorhergehenden Ansprüche, das einen Schritt zur Vorbehandlung des Wärmeträgermediums umfasst, das einen Teilschritt zur Rückgewinnung des flüssigen Wärmeträgermediums am Austritt des Mantels des Rohrbündelreaktors in dem Dehydrierungsschritt und einen Schritt zum Komprimieren und/oder Erwärmen des Wärmeträgermediums zum Erhalten eines Wärmeträgermediums umfasst, das bei der Eintrittstemperatur und dem Eintrittsdruck des Wärmeträgermediums in dem Mantel im Dehydrierungsschritt gasförmig ist.

15. Verfahren zur Herstellung von Butadien aus einem Ethanol-Einsatzmaterial, das mindestens 80 Gewichts-% Ethanol umfasst, umfassend:

A) einen Schritt zur Umsetzung von Ethanol zu Acetaldehyd, bei dem das Verfahren zur Dehydrierung von Ethanol nach einem der Ansprüche 1 bis 14 verwendet wird, wobei das Einsatzmaterial, das den Rohren des Rohrbündelreaktors zugeführt wird, zumindest teilweise ein Anteil eines Austrags mit hohem Ethanolanteil, vorteilhafterweise aus Schritt E1) ist, und so ein Dehydrierungsaustrag produziert wird, und gegebenenfalls einen Trennabschnitt zum Behandeln des Dehydrierungsaustrags und Abtrennen von mindestens einem gasförmigen Wasserstoffaustrag und einem flüssigen Ethanol-/Acetaldehydaustrag; B) einen Schritt zur Umsetzung zu Butadien umfassend zumindest einen Reaktionsabschnitt B, dem zumindest teilweise ein Anteil des Dehydrierungsaustrags aus Schritt A) oder des optionalen Ethanol-/Acetaldehydaustrags aus dem optionalen Trennabschnitt in Schritt A) oder jeweils dieser gesamte Austrag zugeführt wird, gegebenenfalls ein flüssiger Austrag mit hohem Ethanolanteil vorteilhafterweise aus Schritt C1), ein Anteil eines Austrags mit einem hohen Anteil an Acetaldehyd vorteilhafterweise aus Schritt E1) oder dieser gesamte Austrag, der in Gegenwart eines Katalysators, der vorzugsweise das Element Tantal und einen anorganischen Träger, der vorzugsweise Siliciumdioxid umfasst, umfasst, bei einer Temperatur zwischen 300 und 400 °C und einem Druck zwischen 0,1 und 1,0 MPa durchgeführt wird, wobei der Volumenstrom beim Eintrag derart geregelt wird, dass das Molverhältnis von Ethanol bezogen auf Acetaldehyd am Eintritt des Reaktionsabschnitts zwischen 1 und 5 liegt, und einen Trennabschnitt zum Behandeln des Austrags des Reaktionsabschnitts B und Abtrennen mindestens eines gasförmigen Austrags und eines flüssigen Austrags;

C1) gegebenenfalls einen Schritt zur Behandlung von Wasserstoff, der mindestens einen Kompressionsabschnitt umfasst, in dem der Wasserstoffaustrag aus Schritt A) bei einem Druck zwischen 0,1 und 1,0 MPa komprimiert wird, und einen Gas-Flüssigkeit-Waschschritt, dem bei einer Temperatur zwischen 15 °C und -30 °C ein Anteil des Austrags mit hohem Ethanolanteil vorteilhafterweise aus Schritt E1) und ein Anteil des Ethanol-/Acetaldehydaustrags aus Schritt A) zugeführt wird, und bei einer Temperatur zwischen 25 °C und 60 °C der komprimierte Wasserstoffaustrag zugeführt wird und in dem mindestens ein flüssiger Austrag mit einem hohen Ethanolanteil und ein gereinigter Wasserstoffaustrag produziert wird;

D1) einen Schritt zur Extraktion von Butadien, der mindestens Folgendes umfasst:

(i) einen Kompressionsabschnitt, in dem der gasförmige Austrag aus Schritt B) bei einem Druck zwischen 0,1 und 1,0 MPa komprimiert wird, wobei gegebenenfalls der komprimierte gasförmige Austrag aus Schritt B) anschließend auf eine Temperatur zwischen 25 °C und 60 °C abgekühlt wird,

(ii) einen Gas-Flüssigkeit-Waschabschnitt, der eine Waschkolonne umfasst, der am Kopf bei einer Temperatur zwischen 20 und -20°C ein Ethanolstrom, der aus dem Ethanol-Einsatzmaterial des Verfahrens und gegebenenfalls einem Anteil des Austrags mit hohem Ethanolanteil vorteilhafterweise aus Schritt E1) besteht, und am Boden der komprimierte und gegebenenfalls abgekühlte gasförmige Austrag aus Schritt B) zugeführt wird, und in dem mindestens ein flüssiger Waschaustrag und ein gasförmiger Austrag mit Nebenprodukten produziert wird, und

(iii) einen Destillationsabschnitt, der bei einem Druck zwischen 0,1 und 1 MPa betrieben wird und dem zumindest der flüssige Austrag aus Schritt B) und der flüssige Austrag aus dem Gas-Flüssigkeit-Waschabschnitt zugeführt wird und in dem mindestens ein unbehandelter Butadienaustrag und ein Ethanol/Acetaldehyd/Wasser-Austrag produziert wird;

D2) einen Schritt zur ersten Reinigung von Butadien, der mindestens einen Gas-Flüssigkeit-Waschabschnitt umfasst, dem am Boden der unbehandelte Butadienaustrag aus D1) und am Kopf ein Wasserstrom zugeführt wird, der ein Wasserstrom ist, der von außerhalb des Verfahrens zur Herstellung von Butadien stammt, und/oder ein Anteil des Wasseraustrags vorteilhafterweise aus Schritt E1), wobei in dem Waschabschnitt am Kopf ein vorgereinigter Butadienaustrag und am Boden ein Abwasseraustrag produziert wird;

D3) einen weiteren Schritt zur Reinigung von Butadien, dem zumindest der vorgereinigte Butadienaustrag aus Schritt D2) zugeführt wird und in dem mindestens ein gereinigter Butadienaustrag produziert wird;

E1) einen Schritt zur Austragsbehandlung, dem mindestens ein Wasser/Ethanol/Acetaldehyd-Raffinat vorteilhafterweise aus Schritt E2) zugeführt wird und in dem mindestens ein Austrag mit einem hohen Ethanolanteil, ein Austrag mit einem hohen Acetaldehydanteil und ein Austrag mit einem hohen Wasseranteil produziert wird.

E2) einen Schritt zum Beseitigen von Verunreinigungen und braunen Ölen, dem mindestens der Ethanol/Acetaldehyd/Wasser-Austrag aus Schritt D1) und mindestens ein Anteil eines Austrags mit hohem Wasseranteil vorteilhafterweise aus Schritt E1) zugeführt wird und in dem mindestens ein Wasser/Ethanol/Acetaldehyd-Raffinat, ein Austrag mit leichten braunen Ölen und ein Austrag mit schweren braunen Ölen produziert wird;

## Claims

1. Process for the dehydrogenation of ethanol to give acetaldehyde, comprising a stage of dehydrogenation of a feedstock comprising ethanol, said dehydrogenation stage employing a reaction section comprising at least one multitubular reactor which comprises one or a plurality of tubes and a shell,

said tube(s) each comprising at least one fixed bed of at least one dehydrogenation catalyst,
said feedstock feeding said tube(s) in gaseous form, at an inlet temperature of said feedstock of greater than or equal to 240°C, at an inlet pressure of said feedstock of between 0.1 and 1.0 MPa, and at a weight hourly space velocity (WWH) of the feedstock at the inlet of between 2 and 15 h$^{-1}$,
a heat-transfer fluid circulating in said shell so that said heat-transfer fluid is introduced into said shell in gaseous form and is, at the shell outlet, at least partly in liquid form,
said heat-transfer fluid being introduced into the shell at a flow rate such that the ratio of the flow rate by weight of said heat-transfer fluid at the shell inlet, with respect to the flow rate by weight of said feedstock at the inlet of the tube(s), is greater than or equal to 1.0,

said heat-transfer fluid being introduced into the shell at an inlet temperature of the heat-transfer fluid of greater than or equal to 260°C and less than or equal to 400°C, and at an inlet pressure of the heat-transfer fluid of greater than or equal to 0.10 MPa and less than or equal to 1.10 MPa;

said process producing a dehydrogenation effluent comprising at least acetaldehyde, hydrogen and unconverted ethanol.

2. Process according to Claim 1, in which the heat-transfer fluid is an oil comprising an eutectic mixture of organic compounds having saturated vapor pressures such that the difference between the saturated vapor pressures of the organic compounds of the oil, at a given temperature, is less than or equal to 50 Pa, preferably less than or equal to 20 Pa, in a preferred way less than or equal to 10 Pa.

3. Process according to Claim 1 or 2, in which the feedstock comprises at least 50% by weight of ethanol, preferentially at least 70% by weight of ethanol, in a preferred way at least 80% by weight of ethanol, and optionally water, preferably at a content of less than 50% by weight, preferentially of less than 30% by weight, in a preferred way of less than 20% by weight.

4. Process according to one of the preceding claims, in which said at least one multitubular reactor comprises a plurality of tubes, preferably at least 100 tubes, preferentially at least 1000 tubes and in a preferred way at least 2000 tubes, and preferably less than 20 000 tubes, in a preferred way less than 10 000 tubes, for example between 5000 and 6000 tubes.

5. Process according to one of the preceding claims, in which the tube(s) of the multitubular reactor has (have) a length of between 1 and 6 m, in a preferred way between 2 and 3 m.

6. Process according to one of the preceding claims, in which the tube(s) of the multitubular reactor exhibit(s) an internal diameter between 30.0 and 60.0 mm, preferentially between 40.0 and 50.0 mm, and a tube wall thickness preferably between 1.5 and 5.0 mm, preferentially between 2.0 and 4.0 mm and in a preferred way between 2.2 and 3.2 mm.

7. Process according to one of the preceding claims, in which the dehydrogenation catalyst comprises at least the element copper on an inorganic support, preferably silica.

8. Process according to one of the preceding claims, in which the dehydrogenation catalyst is in the form of particles with a mean equivalent diameter between 0.5 and 10.0 mm, preferably between 1.0 and 5.0 mm.

9. Process according to one of the preceding claims, in which the inlet temperature of the feedstock in the tube(s) is between 240°C and 350°C, preferentially between 250°C and 300°C, in a preferred way between 260°C and 290°C.

10. Process according to one of the preceding claims, in which the inlet pressure of the feedstock in the tube(s) is between 0.2 and 0.5 MPa, preferentially between 0.3 and 0.4 MPa.

11. Process according to one of the preceding claims, in which the weight hourly space velocity (WWH) of the feedstock at the inlet of the reactor is between 2 and 10 $h^{-1}$.

12. Process according to one of the preceding claims, in which the heat-transfer fluid is introduced into the shell of the multitubular reactor at a flow rate such that the ratio of the flow rate by weight of said heat-transfer fluid at the shell inlet, with respect to the flow rate by weight of said feedstock at the inlet of the tube(s), is greater than or equal to 1.5 and advantageously less than or equal to 10.0, preferably less than or equal to 5.0, in a preferred way less than or equal to 2.0.

13. Process according to one of the preceding claims, in which the heat-transfer fluid is introduced into the shell of the multitubular reactor at an inlet temperature of greater than or equal to 260°C, preferably of greater than or equal to 270°C, in a preferred way of greater than or equal to 290°C, and of less than or equal to 400°C, preferably of less than or equal to 380°C, and at an inlet pressure of the heat-transfer fluid of greater than or equal to 0.10 MPa, preferably of greater than or equal to 0.13 MPa, in a preferred way of greater than or equal to 0.20 MPa, and of less than or equal to 1.10 MPa, in a preferred way of less than or equal to 0.85 MPa.

14. Process according to one of the preceding claims, comprising a stage of conditioning of the heat-transfer fluid comprising a substage of recovery of the liquid heat-transfer fluid at the shell outlet of the multitubular reactor of the dehydrogenation stage and a stage of compression and/or heating of the heat-transfer fluid in order to obtain a heat-transfer fluid in gaseous form at the inlet temperature and pressure of the heat-transfer fluid in the shell of the dehydrogenation stage.

15. Process for the production of butadiene from an ethanol feedstock comprising at least 80% by weight of ethanol, comprising:

A) a stage of conversion of ethanol into acetaldehyde employing the process for the dehydrogenation of ethanol according to any one of Claims 1 to 14, in which said feedstock which feeds the tubes of the multitubular reactor is at least in part a fraction of an ethanol-rich effluent, advantageously resulting from stage E1), in order to produce a dehydrogenation effluent,
and optionally a separation section in order to treat the dehydrogenation effluent and to separate at least a hydrogen effluent in gaseous form and an ethanol/acetaldehyde effluent in liquid form;
B) a stage for conversion into butadiene comprising at least a reaction section B fed at least with a fraction or all of said dehydrogenation effluent resulting from stage A), or optional ethanol/acetaldehyde effluent resulting from the optional separation section of stage A), optionally with a liquid ethanol-rich effluent advantageously resulting from stage C1), with a fraction or all of an acetaldehyde-rich effluent advantageously resulting from stage E1), operated in the presence of a catalyst, preferably comprising the element tantalum and an inorganic support preferably comprising silica, at a temperature of between 300°C and 400°C and at a pressure of between 0.1 and 1.0 MPa, the feed flow rates being adjusted so that the molar ratio of the ethanol, with respect to the acetaldehyde, at the inlet of said reaction section is between 1 and 5, and a separation section in order to treat the effluent from said reaction section B and to separate at least a gaseous effluent and a liquid effluent;
C1) optionally a stage of treatment of the hydrogen comprising at least a compression section compressing said hydrogen effluent resulting from stage A) to a pressure of between 0.1 and 1.0 MPa and a gas-liquid scrubbing section fed at a temperature of between 15°C and -30°C with a fraction of said ethanol-rich effluent advantageously resulting from stage E1) and with a fraction of said ethanol/acetaldehyde effluent resulting from stage A) and fed at a temperature of between 25°C and 60°C with said compressed hydrogen effluent, and producing at least a liquid ethanol-rich effluent and a purified hydrogen effluent;
D1) a stage of extraction of the butadiene comprising at least:

(i) a compression section compressing said gaseous effluent resulting from stage B) to a pressure of between 0.1 and 1.0 MPa, optionally said compressed gaseous effluent resulting from stage B) subsequently being cooled to a temperature between 25°C and 60°C,
(ii) a gas-liquid scrubbing section comprising a scrubbing column fed, at the top, at a temperature of between 20 and -20°C, with an ethanol flow consisting of the ethanol feedstock of the process and optionally of a fraction of the ethanol-rich effluent advantageously resulting from stage E1) and, at the bottom, with said gaseous effluent resulting from stage B) compressed and optionally cooled, producing at least a liquid scrubbing effluent and a gaseous by-products effluent, and
(iii) a distillation section operated at a pressure of between 0.1 and 1 MPa, fed at least with the liquid effluent resulting from said stage B) and with the liquid effluent from said gas-liquid scrubbing section, producing at least a crude butadiene effluent and an ethanol/acetaldehyde/water effluent;

D2) a stage of first purification of the butadiene comprising at least a gas-liquid scrubbing section fed at the bottom with the crude butadiene effluent resulting from D1) and at the top with a flow of water which is a flow of water of origin external to said process for the production of butadiene and/or a fraction of the aqueous effluent advantageously resulting from stage E1), said scrubbing section producing a pre-purified butadiene effluent at the top and a waste water effluent at the bottom;
D3) a subsequent stage of purification of the butadiene, fed at least with said pre-purified butadiene effluent resulting from said stage D2) and producing at least a purified butadiene effluent;
E2) a stage of removal of impurities and brown oils, fed at least with the ethanol/acetaldehyde/water effluent resulting from stage D1) and with at least a fraction of a water-rich effluent advantageously resulting from stage E1), and producing at least a water/ethanol/acetaldehyde raffinate, a light brown oils effluent and a heavy brown oils effluent;
E1) a stage of treatment of the effluents which is fed at least with a water/ethanol/acetaldehyde raffinate advantageously resulting from stage E2), and producing at least an ethanol-rich effluent, an acetaldehyde-rich effluent and a water-rich effluent.

## RÉFÉRENCES CITÉES DANS LA DESCRIPTION

**Documents brevets cités dans la description**

- US 5227563 A
- WO 2018046515 A
- FR 3089973
- FR 3090632
- FR 3026100

**Littérature non-brevet citée dans la description**

- **FILHO R.M.** A multitubular reactor for obtention of acetaldehyde by oxidation of ethyl alcohol. *Chemical Engineering Science*, 01 June 1992, vol. 47 (9-11), 2571-2576
- Les Biocarburants, État des lieux, perspectives et enjeux du développement. **DANIEL BALLERINI**. Technip. 2006